# EUROPEAN PATENT APPLICATION

(11) **EP 2 319 821 A1**
(43) Date of publication of application: **11.05.2011**
(21) Application number: 09175328.5
(22) Date of filing: 06.11.2009
(51) Int. Cl.: C07C 7/20, C07C 13/39, C23C 16/06, H01L 21/312

(54) **Stabilization of bicycloheptadiene**

(71) Applicant: L'Air Liquide Société Anonyme pour l'Etude et l'Exploitation des Procédés Georges Claude, 75007 Paris (FR)
(72) Inventor: McAndrew James, J.F., Chadds Ford, PA 19317 (US); Girard, Jean-Marc, 75009 Paris (FR); Marot, Yves, 78530 Buc (FR); Gobard, Daniel, André, 75013 Paris (FR)
(74) Representative: Conan, Philippe Claude

(57) **Abstract**

Disclosed are stabilized bicyclo[2.2.1]hepta-2,5-diene compositions and methods of making and using the same.

## Description

Insulating films that simultaneously provide adequate mechanical strength with low dielectric constant ("low-k") are required for semiconductor manufacturing (see for example International Technology Roadmap for Semiconductors, 2007 edition). In recent years, the most successful materials have been carbon-doped silicon oxides containing Si, C, O, and H (i.e. "SiCOH"), deposited by Plasma-Enhanced Chemical Vapor Deposition (PECVD), as described for example in US 7,030,468 and US 7,282,458 (both Gates et al.).

A major advantage of these materials is that they can be deposited using vapor deposition equipment similar to that in general use for depositing Si0₂ dielectrics. Vapor deposition processing may be performed in a high purity, vacuum environment in which air is rigorously excluded from all of the reagents present. For example, it is common to use a nitrogen carrier gas with a specification of less than 10 parts per billion, or even less than 1 part per billion, of oxygen.

The dielectric constant of an insulating film may be further reduced by using a porogen to introduce porosity, as in, for example US 7,288,292 (also Gates et al.). An example of a porogen is bicyclo[2.2.1]hepta-2,5-diene, also called 2,5-norbornadiene, referred to here as BCHD, as shown.

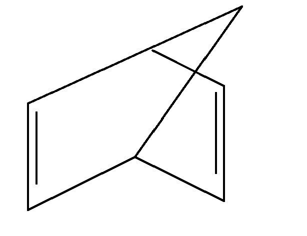

Use of BCHD as a porogen was described in US 6,312,793 (Grill et al), and has been identified as a "best-known-method", for introducing porosity to an insulating film. However, BCHD is a highly reactive olefinic liquid which, in the absence of an appropriate inhibitor, has been observed to self-polymerize. See US 3,860,497 and US 3,140,275. Self-polymerization is believed to proceed more rapidly as temperature is increased.

A common method to deliver BCHD to a vapor deposition process is to deliver a stream of liquid BCHD via a heated "injection valve", which vaporizes the liquid as it passes through a variable opening, and combines the vaporizing liquid flow with a controlled flow of carrier gas. The flow of BCHD vapor is controlled by adjusting the variable opening. As can be appreciated, the presence of oligomers of low volatility easily leads to accumulation of these low volatile materials in the opening, ultimately causing clogging. In less severe cases, oligomers can be entrained as liquid droplets in the gas stream, leading to nonuniform delivery of porogen to the process.

Oligomers formed in BCHD during storage are frequently soluble in BCHD and thus are carried with the BCHD stream to the vaporizer, resulting in the difficulties outlined above. In addition, because the vaporizer is heated, BCHD oligomers may be formed in the vaporizer itself. This is particularly the case if a sample of BCHD is allowed to stand in the vaporizer without flowing (during an interruption in production, for example).

Therefore there is a need to prevent polymerization of BCHD during both storage and the higher temperature conditions found during vapor deposition.

Numerous patents and some publications describe the use of various inhibitors to prevent the polymerization of various compounds, particularly olefinic compounds. In section 5.4 of *The Chemistry of Free Radical Polymerization,* Moad and Solomon disclose that stable radicals, captodative olefins, phenols, quinones, oxygen, and certain transition metal salts are common inhibitors (Elsevier Science Ltd. 1995). In general, radical species (i.e. species with an unpaired electron) are known to initiate polymerization by reacting with olefins such as BCHD. The reaction product is another radical which propagates the chain of polymerization. Inhibitors react with radicals to form non-reactive products and therefore prevent initiation.

The role of oxygen in initiation and inhibition of polymerization has been discussed at page 262 of Moad and Solomon and in Principles of Polymerization, George Odian, 1991 (Wiley) p. 264. Oxygen is known to convert free radicals to peroxides, which are less reactive, thereby inhibiting polymerization. Therefore, oxygen must often be excluded when polymerization is desired, such as in the manufacture of plastics. However, the presence of peroxides is not acceptable if long-term resistance to polymerization is needed, because peroxides decompose to re-form free radicals. Decomposition occurs slowly at ambient temperatures, but more quickly when heated. Peroxides can also be formed by the reaction of oxygen with relatively non-reactive organic molecules, which leads to an eventual increase in radical concentration when those peroxides decompose. In this way, oxygen may also act as an initiator of polymerization.

For BCHD, it has been found that oxygen infiltration into the system (via leaks, for example) generally leads to formation of non-volatile oligomers and the consequent problems discussed previously. This may be attributed to peroxide formation as discussed above. Therefore it is important that BCHD be kept oxygen-free.

BCHD is commercially available containing a phenolic inhibitor: 2,6-di-tert-butyl-4-methylphenol (also called BHT) (see for example product no. B33803 in the Sigma-Aldrich catalog). The class of "phenolic inhibitors" (also known as hydroquinones) is described by Moad and Solomon as "commonly added to many commercial monomers to prevent polymerization during transport and storage." ld at p. 263. BHT and other phenolic inhibitors are known to be more effective in inhibiting polymerization of olefinic compounds in the presence of air or oxygen. See, e.g., Introduction Kurland, J. Polym. Sci, 18 1139-1145 (1980); Why Oxygen?, Levy, Plant/Operations Progress, 6 188 (1987); p. 37, top of second column, Gustin, Chemical Health and Safety, Nov/Dec. 2005; and Nicolson, Plant /Operations Progress, Vol. 10(3) p 171-183 (1991).

In US 2007/0057235 (Teff et al.) stabilized BCHD compositions using phenolic inhibitors other than BHT are disclosed. Formation of "highly soluble, low volatility solid products" in BCHD is attributed to the "presence of adventitious air". Performance data is provided in terms of residue observed following air exposure. However, as can be understood from the preceding discussion, data obtained in the presence of air is not the most relevant for semiconductor manufacturing applications, and there remains a need for a BCHD composition that remains free of oligomers even in the absence of air.

Another group of stabilizers/inhibitors frequently used with olefinic compounds is stable nitroxides, such as 2,2,6,6-tetramethyl-piperindino-1-oxy or 2,2,6,6-tetramethyl-1-piperidinyloxyl (TEMPO), as shown.

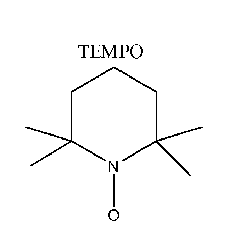

Moad and Solomon list TEMPO as the most reactive inhibitor for carbon-centered radicals.

US 4,670,131 (Ferrell) discloses that t]he use of stable nitroxides and other stable free radicals and precursors thereto are well documented in the patent and open literature as stabilizers for olefinic organic compounds. The prior art teaches that these stable free radicals are useful for the prevention of premature radically induced polymerization of the olefinic monomer during storage and as antioxidants. Ferrell further discloses that the cited compounds, including TEMPO, control fouling in processing equipment, such as equipment used for heating and vaporizing, for hydrocarbon streams containing olefinic organic compounds having 2 to 20 carbon atoms, including butadiene and isoprene.

US 5,616,753 (Turner et al.) discloses an inhibited silane composition comprising a polymerizable silane and a non-aromatic stable free radical, such as TEMPO, in an amount sufficient to inhibit polymerization of the silane during production, purification, and in situ prior to application, with or without the presence of oxygen. Non-silane compounds are not addressed. Turner et al. disclose use of the non-aromatic stable free radicals as both an inhibitor and a short stopping agent, an agent that may be added to stop polymerization after it has started.

US 5,880,230 (Syrinek) discloses TEMPO as a short stopping agent for emulsion polymerization of vinyl or diene monomers, including 1, 3-butadiene and isoprene. In the background Syrinek acknowledges that stable nitroxyl free radicals are known to be used at very low concentrations to inhibit polymerization of vinyl monomers during manufacture, purification, storage, and transport, where polymerization is due to incidental free radicals from heat or oxygen.

US 6,686,422 and US 6,525,146 (Shahid) describe the inhibition of "popcorn" polymer growth during the distillation of diene compounds, such as butadiene and isoprene, using the combination of a hindered or unhindered phenol and a stable nitroxide. BHT and MHQ (MethoxyHydroQuinone) are examples of unhindered phenols. An example of a stable nitroxide is TEMPO. Shahid mentions that up to about 10,000 ppm of phenol and up to about 10,000 ppm of stable nitroxide may be used, with the preferable concentration range being up to about 5,000 ppm of phenol and up to about 400 - 500 ppm stable nitroxide. Shahid discloses that popcorn polymerization may occur in both the gaseous and liquid phase, and is more likely to occur when the temperature is high.

WO 2007/045886 (Loyns et al.) discloses the use of specific stable nitroxides and mixtures of stable nitroxides that are effective in both a water phase and organic phase for stabilization of "ethylenically unsaturated monomers" during manufacture and purification of the monomers. Examples of the monomers include styrene, α-methylstyrene, styrene sulphonic acid, vinyl toluene, divinylbenzene, and dienes such as butadiene or isoprene. The specific stable nitroxides claimed are of the formula shown, 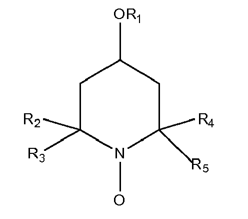 where R₁ is C₄-C₂₀ hydrocarbyl and R₂ _ R₅ are each independently C₁-₈ alkyl. The use of other stable nitroxides (such as TEMPO) in combination with stable nitroxides of the indicated formula is also claimed.

US 2009/0159843 and US 2009/0159844 (both to Mayorga et al.) disclose stabilized compositions consisting essentially of unsaturated hydrocarbon-based precursor materials, such as BCHD or isoprene, and a stabilizer selected from either a hydroxybenzophenone or a nitroxyl radical, such as TEMPO. However, after disclosing the use of 20 ppm to 200 ppm TEMPO in the initial application filed February 27, 2008 (see paragraph 0057), Mayorga et al. filed a second application on May 28, 2008 directed to the same disclosure, but indicating in Examples 33-41 that 100 ppm TEMPO is not sufficient to address dynamic stability during direct liquid injection at industry relevant conditions and claiming a range of 1,000 ppm to 5,000 ppm.

While the use of nitroxyl radicals such as TEMPO as polymerization inhibitors is well-known, nitrone compounds are primarily used as "spin-traps" to elucidate reaction mechanisms, most notably using electron-paramagnetic-resonance spectroscopy, as discussed in Moad and Solomon, p.265. Inhibition of photopolymerization using nitrone compounds has been described in US 6,162,579 (Stengel et al). The polymerizing compounds in question are ethers, esters, and partial esters of acrylic and methacrylic acid.

Based on the literature, it appears that TEMPO and related stable nitroxyl radicals are a preferred choice for stabilizing olefinic compounds. However, as implemented by Mayorga et al. relatively high concentrations (> 1000 ppm) of TEMPO in BCHD have been found necessary to prevent deposition in vaporizers used in the semiconductor industry. Even with 1800ppm TEMPO added, the longest duration of continuous flow cited by Mayorga et al. as giving no residue on a vaporizer was 10 hours, which is very short for practical manufacturing. No means is given for predicting the conditions needed for avoiding residue accumulation during longer-term use.

Lower concentrations of inhibitor are inherently desirable in precursors used for semiconductor manufacturing, where high purity materials are required. In particular, nitrogen-containing inhibitors are preferably used at lower concentrations, because nitrogen compounds are known to cause photoresist poisoning.

It is preferable to avoid nitrogen altogether by using nitrogen-free inhibitors. One such class is the phenolic inhibitors, as discussed by Teff in US 2007/0057235. However, as discussed above, it is well-known that phenolic inhibitors work best in the presence of oxygen, which must be carefully excluded from BCHD.

Another class of nitrogen-free inhibitors is the quinones, for example p-benzoquinone. Moad and Solomon disclose that p-benzoquinone is approximately 100 times less effective than TEMPO as an inhibitor. On page 259 of the third edition of Principles of Polymerization, George Odian discloses that benzoquinone acts as an inhibitor in styrene polymerization until it is consumed (John Wiley & Sons, Inc. 1991 ). Odian also provides a graphical representation of the percent polymerization conversion of styrene at 100°C versus time and includes a plot for 0.1 % benzoquinone. On page 264, Odian discloses that benzoquinone acts an inhibitor (i.e. prevents polymerization for a period of time) for styrene and vinyl acetate, which are characterized as having electron-rich propagating radicals, but only acts as a retarder (i.e. slows polymerization, but does not prevent it) for acrylonitrile and methyl methacrylate, which have electron-poor propagating radicals. Based on this information, and as BCHD is neither electron-rich nor electron-poor, benzoquinone is expected to be of intermediate effectiveness in inhibiting/retarding the polymerization of BCHD. Yassin and Rizk (J. Polymer Sci: Polymer Chemistry Edition (16) 1475-1485 (1978) "Charge-Transfer Complexes as Polymerization Inhibitors: l. Amine-Chloranil Complexes as Inhibitors for the Radical Polymerization of Methyl Methacrylate") have described how N,N-dimethylaniline and triethylamine may be used to enhance the inhibition of methyl methacrylate polymerization by chloranil (also called tetrachlorobenzoquinone). This inhibition enhancement is described as specific to electron-poor monomers.

In US 5,840,976 (Sato et al.), benzoquinone or alkali-modified derivatives of a quinone are used during distillation to stabilize N-vinylamides, including N-vinylformamide and N-vinylacetamide. Sato et al. disclose that the n-vinylamide compounds are very reactive and may readily be decomposed or polymerized. Table 1 shows decomposition test results comparing n-vinylformamide alone or combined with benzoquinone, anthraquinone, hydroquinone, and hydroquinone monomethyl ether stabilizers, amongst others. A comparison to TEMPO was not provided. Additionally, Sato discloses that 50 ppm to 10,000 ppm of quinone may be used, with smaller amounts ineffective and larger amounts possibly resulting in saturation of the stabilizing effect. In his examples, Sato et al. use 500 ppm and 3000 ppm benzoquinone, achieving better results with 3000 ppm. Sato et al. does not address application of benzoquinone to olefinic compounds other than vinylamides.

The concentration of non-volatile material dissolved in BCHD is clearly useful to assess the likelihood that a given sample of BCHD will be incompletely vaporized or cause vaporizer clogging. Simple a priori calculations indicate that extremely low concentrations of non-volatile material are desirable. For example, consider a standard production situation where BCHD is consumed at a rate on the order of 1g per minute, which implies monthly consumption on the order of 10kg. A vaporizer with a small orifice may be adversely affected by a deposit as small as 1 mg. Assuming that all non-volatile material in the sample may eventually be trapped in the vaporizer leads to a desired concentration of non-volatile material on the order of approximately 0.1 ppbw, provided one is willing to clean or replace the vaporizer monthly. In practice, preparation and analysis of samples of BCHD with such low concentrations of non-volatile material would be prohibitively expensive and impractical for manufacturing. Therefore there is a need to determine whether BCHD samples with non-volatile material greater than these ideal levels may yet successfully be used in manufacturing.

Applicants have identified levels of non-volatile material in stabilized BCHD required for successful implementation in manufacturing. Contrary to the disclosures above, applicants have discovered that several inhibitors, including nitrones, quinones, and mixtures of quinones and stable nitroxides have been found effective to inhibit polymerization of BCHD, even at relatively low concentrations.

Disclosed in the present invention are compositions for providing a low-k film comprising bicyclo[2.2.1]hepta-2,5-diene (BCHD) and at least one additive. The composition contains less than 50 ppmw non-volatile material. The additive is selected from the group consisting of nitrones, quinones, mixtures of nitrones and quinones, mixtures of nitrones and stable nitroxides, and mixtures of quinones and stable nitroxides. The composition may further comprise an electron donor species when the additive comprises a quinone. The selected additive exhibits a vapor pressure ranging from approximately 0,05^{*}133 Pa to approximately 50^{*}133 Pa at 20°C, and more preferably from approximately 0,08^{*}133 Pa to approximately 10^{*}133 Pa at 20°C. One preferred additive is 5,5-dimethyl-1-pyrroline N-oxide at concentrations between approximately 300 ppmw and approximately 1,000 ppmw. A second preferred additive is benzoquinone at concentrations between approximately 100 ppmw to approximately 500 ppmw. Another preferred additive is benzoquinone combined with 2,2,6,6-tetramethyl-1-piperidinyloxyl (TEMPO) at concentrations ranging between approximately 1 ppmw to approximately 150 ppmw and between approximately 100 ppmw to approximately 200 ppmw, respectively. The compositions may be used to lower the dielectric constant of an insulating film, such as carbon doped silicon oxides, used in the manufacture of semiconductor materials, photovoltaic, LCD-TFT, catalysts, or flat panel-type devices.

Also disclosed is a method for inhibiting polymerization in BCHD, thus making its use in semiconductor processing, and particularly its delivery via a vaporizer, much more convenient. BCHD is stabilized by adding at least one additive selected from the group consisting of nitrones, quinones, mixtures of nitrones and quinones, and mixtures of quinones and stable nitroxides. An electron donor species may be combined with the quinone prior to its addition to the BCHD. The stabilization works in the absence of air and at high temperature (i.e. 80°C and above). The additive may be added within one hour of purification of BCHD. The BCHD is preferably purified to at least 99.0%. The additive may be added to an evaporation vessel or a condensation vessel during purification. One preferred additive is 5,5-dimethyl-1-pyrroline N-oxide at concentrations between approximately 300 ppmw and approximately 1,000 ppmw. A second preferred additive is benzoquinone at concentrations between approximately 100 ppmw to approximately 500 ppmw. Another preferred additive is benzoquinone and 2,2,6,6-tetramethyl-1-piperidinyloxyl (TEMPO) at concentrations ranging between approximately 1 ppmw to approximately 150 ppmw and between approximately 100 ppmw to approximately 200 ppmw, respectively.

Also disclosed is a method for forming a composition for providing a low-k film by combining BCHD with at least one additive selected from the group consisting of nitrones, quinones, mixtures of nitrones and quinones, mixtures of nitrones and stable nitroxides, and mixtures of quinones and stable nitroxides. The composition may further include an electron donor species when the additive comprises a quinone. The BCHD and additive are preferably combined within one hour of purification of BCHD. The BCHD is preferably purified to at least 99.0%. The additive may be added to an evaporation vessel or a condensation vessel during purification. One preferred additive is 5,5-dimethyl-l-pyrroline N-oxide at concentrations between approximately 300 ppmw and approximately 1,000 ppmw. A second preferred additive is benzoquinone at concentrations between approximately 100 ppmw to approximately 500 ppmw. Another preferred additive is benzoquinone and 2,2,6,6-tetramethyl-1-piperidinyloxyl (TEMPO) at concentrations ranging between approximately 1 ppmw to approximately 150 ppmw and between approximately 100 ppmw to approximately 200 ppmw, respectively.

Also disclosed is a method of forming an insulating film layer on a substrate by providing a reaction chamber having at least one substrate disposed therein, introducing at least one precursor compound into the reaction chamber, introducing into the reaction chamber a composition comprising bicyclo[2.2.1]hepta-2,5-diene and at least one additive selected from the group consisting of nitrones, quinones, mixtures of nitrones and quinones, mixtures of nitrones and stable nitroxides, and mixtures of quinones and stable nitroxides, and contacting the at least one precursor compound, the composition, and the substrate to form an insulating film on at least one surface of the substrate using a deposition process. The composition may further include an electron donor species when the additive comprises a quinone. The composition is vaporized at a temperature between about 70°C and about 110°C in the presence of a carrier gas prior to introduction into the reaction chamber. One preferred additive is 5,5-dimethyl-1-pyrroline N-oxide at concentrations between approximately 300 ppmw and approximately 1,000 ppmw of 5,5-dimethyl-1-pyrroline N-oxide. A second preferred additive is benzoquinone at concentrations between approximately 100 ppmw to approximately 500 ppmw. Another preferred additive is benzoquinone and 2,2,6,6-tetramethyl-1-piperidinyloxyl (TEMPO) at concentrations ranging between approximately 1 ppmw to approximately 150 ppmw and between approximately 100 ppmw to approximately 200 ppmw, respectively.

Certain abbreviations, symbols, and terms are used throughout the following description and claims and include: The amount of non-volatile material is the ratio of residue weight out of total product weight after complete evaporation at 80°C and atmospheric pressure of a sample of at least 100g; the abbreviations "BCHD" and "NBDE" both refer to bicyclo[2.2.1]hepta-2,5-diene, which is also known as 2,5-norbornadiene; the abbreviation "BHT" refers to 2,6-di-tert-butyl-4-methylphenol; the abbreviation "MHQ" refers to 4-methoxyphenol; the abbreviation "PBN" refers to N-tert-butyl-a-phenylnitrone; the abbreviation "DMPO" refers to 5,5-dimethyl-1-pyrroline-N-oxide; the abbreviation "ppm" refers to parts per million; the abbreviation "ppmw" refers to parts per million by weight; the term "additive" and "inhibitor" collectively refer to the compound used to prevent polymerization of BCHD; the abbreviation "slm" refers to standard liters per minute; the abbreviation "GC-FID" refers to gas chromatography - flame ionization detection and all percentages recited have been calculated from GC-FID analysis; the abbreviation "MIM" refers to Metal Insulator Metal (a structure used in capacitors); the abbreviation "DRAM" refers to dynamic random access memory; the abbreviation "FeRAM" refers to ferroelectric random access memory; the abbreviation "CMOS" refers to complementary metal-oxide-semiconductor; the abbreviation "UV" refers to ultraviolet; the abbreviation "RF" refers to radiofrequency; the abbreviation "cc" refers to a cubic centimeter and is interchangeable with mL, or milliliter; the abbreviation "scc" refers to "standard cubic centimeter" which is the quantity of gas or vapor occupying one cc under standard conditions of 0°C and 1 bar pressure; the abbreviation "scc/min" and "sccm" both refer to scc per minute; the term "residue" refers to the material remaining after evaporation of a substance or mixture; and the term "non-volatile materials" refers to materials that do not evaporate under conditions normally used in vaporizers, and includes materials suspended or dissolved in solution or in droplets entrained in a gas stream.

### Brief Description of the Drawing:

For a further understanding of the nature and objects of the present invention, reference should be made to the following detailed description, taken in conjunction with the accompanying drawing, and wherein:

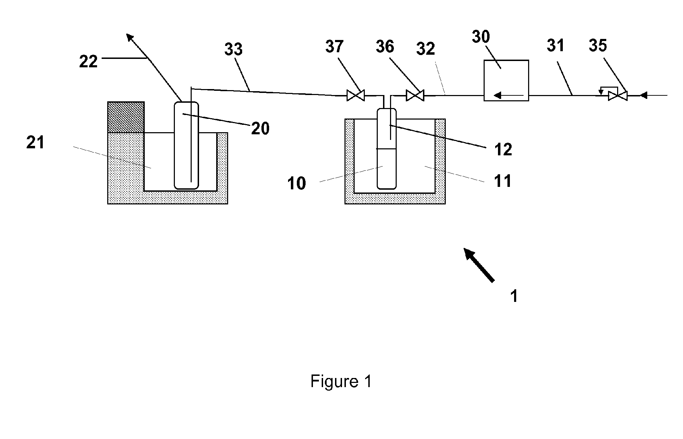

The figure 1 illustrates an evaporation apparatus used to purify BCHD.

Disclosed are compositions for providing a low-k film, methods for stabilizing BCHD, and methods of forming an insulating film layer on a substrate. The composition contains less than 50 ppmw non-volatile material. Nitrones and quinones, alone or in combination with other known inhibitors such as stable nitroxides, have been found effective to inhibit polymerization of BCHD at relatively low concentrations. The BCHD/inhibitor combination may then be vaporized and deposited to form an insulating film on a substrate without the attendant incomplete vaporization and clogging issues experienced in the prior art.

### Nitrones

Nitrones have the general structure illustrated below, in which R¹ to R³ may be hydrogen or substituted or unsubstituted alkyl or aryl groups. The structures for non-limiting examples of nitrones are also provided.

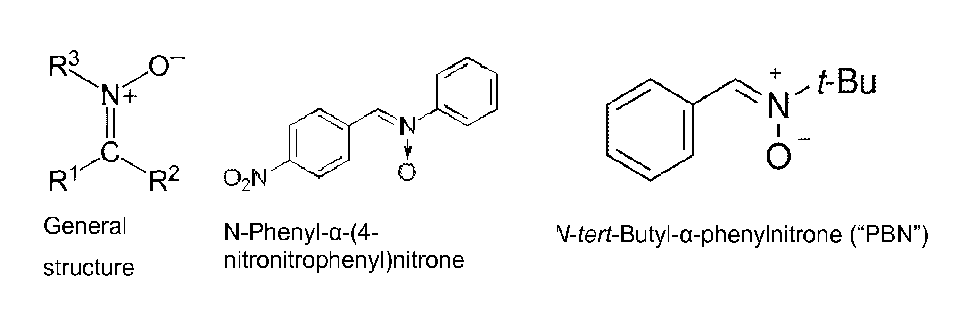

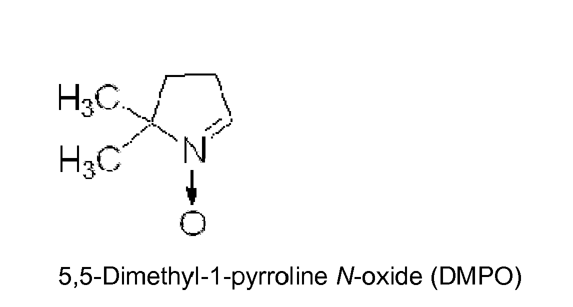

Nitrones are known as "spin traps", used in fundamental chemical studies for their property of reacting with radicals to form stable radical products. As noted in the background, nitrones have been used to inhibit polymerization of photopolymerizable compositions (by Stengel). Nitrones have not previously been used to inhibit polymerization of single components, such as BCHD. Applicants found PBN to be effective in inhibiting the polymerization of BCHD. However, PBN is of low volatility and, upon evaporation of the mixture, leads to a residue due to the PBN itself. PBN may be useful in applications where BCHD is not delivered as a vapor, for example in liquid deposition applications. DMPO is more volatile, having a vapor pressure of 0.4^{*}133 Pa at 75°C, and has been found to lead to lower residues on evaporation than PBN.

### Quinones and Related Compounds

Quinones have the structure, shown at left, where R1 to R4 may be independently H, chlorine, an alkyl group, or a substituted alkyl group. Examples of quinones include p-benzoquinone, duroquinone, 2,5-dichloro-benzoquinone, 2,6-dichlorobenzoquinone and chloranil (aka 2,3,5,6-Tetrachloro-1,4-benzoquinone).

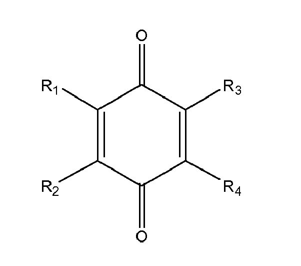

The vapor pressure of benzoquinone (in which R1 to R4 is H) is approximately 0.09^{*}133 Pa at 25°C and its boiling point (at 1 atmosphere) is reported to be 180°C.

Quinones react with radicals to produce radicals in which the unpaired electron is delocalized over the ring structure and the oxygen atoms. The radical formed is stabilized by delocalization and also by some aromatic character. Thus the reactivity of radicals produced is reduced by benzoquinone in a manner somewhat similar to that observed for nitrones.

The effectiveness of quinones in inhibiting polymerization may be enhanced by providing an electron donor species, such as ammonia NH₃ or an amine NR₃ where each R may independently be H, an alkyl group or an aryl group. Two R groups may also be combined to form a cyclic amine. Tertiary amines are preferred to minimize extraneous reactions. Examples of suitable amines include trimethyl amine, triethyl amine, tripropyl amine, tributyl amine, dimethyl aniline, and methyl pyrrolidine. The electron donor species may preferably be combined with the quinone prior to combining the quinone with BCHD or may alternatively be combined with the BCHD/quinone composition.

### Stable Nitroxides

Stable nitroxides have the piperidine-1-oxide structure shown below, in which Y may be hydrogen, oxygen (with double bond), an amino group, a cyano group, a nitro group, an alkoxy group, an alkyl group, an amidogroup (e.g. 4-acetaminodo or 4-maleimidoTEMPO), a carboxylic acid group or an ester group and R1 to R4 may each individually be hydrogen, an alkyl group, or an alkoxy group. Less preferred but still usable Ys include halogen-substituted alkyl or alkoxy groups, thiocyanate groups, sulfonate, and alkylsulfonate groups. An example of a stable nitroxide is TEMPO, illustrated below. The vapor pressure of TEMPO at 20°C is approximately 0.4^{*}133 Pa. A substituted TEMPO having a suitable vapor pressure may also be used. TEMPO and p-hydroxyTEMPO are the most preferred molecules of this class.

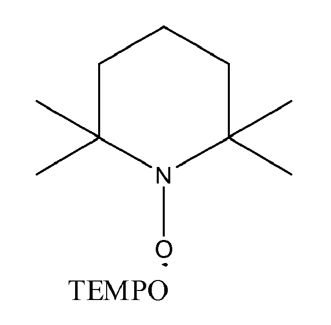

As stable nitroxides are radicals (i.e. having an unpaired electron represented by the dot adjacent the oxygen atom in the illustrations above), they react with other radicals to form non-radical species. Thus stable nitroxides completely remove radicals, rather than merely reacting with them to form less reactive radicals, and are documented in the literature as being highly effective in inhibiting polymerization.

### Volatility Criteria

Any additive selected must exhibit a sufficient vapor pressure to prevent its accumulation in the vaporizer. If the additive exhibits an insufficient vapor pressure, it will accumulate in the vaporizer as a residue, resulting in the same problems caused by BCHD alone.

Inhibitors with too high a vapor pressure are also undesirable, as they will vaporize more rapidly than BCHD, which will eventually result in insufficient inhibitor in BCHD to prevent its polymerization. The vapor pressure of BCHD is about 50^{*}133 Pa at 20°C and 550^{*}133 Pa at 80°C. Too high a vapor pressure is less of an issue in practice as BCHD is more volatile than most candidate inhibitors. In general, inhibitors with vapor pressure in the range from approximately 0.05*133 Pa to approximately 50*133 Pa at 20°C are preferred, with a more preferred range being approximately 0.08*133 Pa to approximately 10*133 Pa at 20°C. As vapor pressure data are often not available at every temperature, the following ranges may also be used: 0.1*133 Pa to 1000*133 Pa at 80°C, more preferably, 0.2*133 Pa to 550*133 Pa at 80°C.

In addition to vapor pressure, volatility may also be determined by the additive's boiling point. A preferred boiling point range at ambient pressure is less than approximately 300°C, more preferably less than approximately 200°C.

The resulting BCHD/additive composition will have less than 50 ppmw non-volatile material, and more preferably less than 20 ppmw. The amount of non-volatile material may be determined by evaporating the BCHD/additive composition and determining the weight of non-volatile material remaining. In one exemplary evaporation process, approximately 150 mL of distilled BCHD having a purity of 99% or higher is mixed with a suitable amount of additive and added to the heated vessel 10 of apparatus 1 of figure 1. Nitrogen having a flow rate of about 0.3 to about 1 slm passes through the apparatus 1. The heated vessel 10 is maintained at a temperature between about 50°C to about 90°C by a silicone oil bath 11. Cryostat 21 maintains the cooled vessel 20 at a temperature between about -25°C to about 25°C. The total amount of non-volatile material in ppmw reflects the amount of non-volatile material remaining in the heated vessel 10 after approximately 3 hours.

### Preferred inhibitor concentration

Concentrations of 300 ppmw of DMPO, of 150 ppmw of benzoquinone, and of a mixture of 150 ppmw benzoquinone and 150 ppmw TEMPO have been found sufficient to stabilize BCHD.

During storage at room temperature, no consumption of benzoquinone has been observed. However, if BCHD is exposed to elevated temperatures, consumption of benzoquinone was observed. A test was carried out as follows: a sample of BCHD stabilized with benzoquinone at 150 ppmw was immersed in an oil bath at 80°C for approximately 7 days. It was then removed and the benzoquinone concentration measured by GC-FID. The results are summarized in the following table:

| | Initial | After 4.6 days at 80°C | After 7.5 days at 80°C | After 72 hours at room temperature |
|---|---|---|---|---|
| Benzoquinone Concentration (ppmw) | 150 | 120 | 60 | 150 |

The results clearly show that benzoquinone is consumed much more quickly at 80°C than at room temperature. Seven days exposure to 80°C is unlikely to occur in normal handling. Thus benzoquinone concentration in the approximately 100-200 ppmw range is preferred to provide an ample safety margin for thermal excursions that might occur during normal handling and subsequent vapor deposition.

The optimum concentration for other inhibitors may be determined by routine experimentation. While differences may be observed between inhibitors, in general it is recommended that when a single inhibitor is used, it should be present at between about 100 ppmw and about 500 ppmw. If more than one inhibitor is used, the total inhibitor concentration should be between about 100 ppmw and about 500 ppmw. Concentrations below 100 ppmw are also effective, but leave the composition vulnerable to deterioration in case of accidental exposure to adverse conditions, such as high temperatures, which cause the inhibitor to be consumed. In the event that the inhibitor is completely consumed, polymerization will proceed much more rapidly.

Too high a concentration of inhibitor should be avoided. At sufficiently high concentrations, impact on the deposited film will occur. In addition, an increase in residue on evaporation has occasionally been observed at high inhibitor concentration (namely, about 1000 ppm) compared to samples with lower inhibitor concentration. While these results are not fully understood, they may be attributable to copolymerization of the inhibitor with BCHD or action of the intended inhibitor as an initiator, similar to the behavior described for oxygen.

### Purification

BCHD must be purified prior to the vapor deposition process. Oligomers of BCHD are often soluble in BCHD. Thus a sample of BCHD that appears clear to the eye may contain a significant fraction of oligomers that remain behind on evaporation of the pure compound, resulting in residue formation in the vaporizer. Metal contaminants may form complexes and may therefore also be present in solution. Metal contaminants may initiate or catalyze polymerization, especially on heating. Therefore it is important that BCHD be carefully purified in order to minimize the formation of non-volatile material. Preferably the BCHD starting material will have a purity of at least 95% and purification will bring this BCHD starting material to a purity of at least 99.0%. BCHD may be supplied with BHT (see for example product no. B33803 in the Sigma-Aldrich catalog). As BHT is not very volatile, purification removes BHT from the starting material.

The purity of BCHD may be determined by gas chromatography using a flame-ionization detector ("GC-FID"). It should be understood that gas chromatography does not detect non-volatile material, which will not pass through the analytical column in the gas phase. Nonetheless, GC-FID is a useful technique for monitoring the overall effectiveness of purification by measuring the level of volatile contaminants present in the BCHD product. The column used herein was a HP-5MS column (30m long, 0.32mm lD, 0,25 micron film thickness). The GC-FID was operated under the following conditions: 1cc/min He carrier gas, 1:100 split; column temperature program: 5 min at 60°C, then 2°C/min up to 80°C, then 10°C/min up to 300°C; and injection temperature: 200°C. One of ordinary skill in the art would recognize that other columns and conditions may also be utilized to determine the BCHD purity.

Proper precautions taken during purification and handling of the purified product are particularly important. The system and product containers must be carefully cleaned and dried before use. Great care must be taken to avoid leaks, to use an atmosphere which is free of moisture and other contaminants such as hydrocarbons, to avoid exposure to air and light, to maintain proper cleanliness of the apparatus, and to introduce inhibitors to the purified BCHD during or as soon as possible after purification is complete, preferably within less than one hour, and more preferably by having the inhibitor already present in the collection vessel. The inhibitor may also be added to the BCHD starting material in the heating vessel of either purification unit (i.e. the distillation or evaporation apparatus). The inhibitor should be well-mixed with the purified BCHD, for example by predissolving the inhibitor in a small amount of BCHD or other solvent. Exposure to particulate contamination must be minimized in preparing the purification system and storage canisters. Particles, especially metal particles, may act as initiators of polymerization.

As oligomerization can be initiated by light exposure, the distillation and/or evaporation apparatus should be constructed from materials that do not transmit light, or, if light-transmissive materials are used, they should be wrapped with opaque material such as metal foil to prevent light transmission. This precaution is particularly important for the portions of the apparatus containing purified material.

BCHD may be purified by distillation, using conventional means to separate both light and heavy contaminants.

BCHD may alternatively be purified by evaporating a sample slowly over several hours under a flowing inert gas, such as but not limited to nitrogen, in a heated vessel 10, having dip tube 12 through which the inert gas flows, and collecting the purified condensate in a cooled vessel 20, as illustrated in figure 1. An oil bath 11 maintains the heated vessel 10 at up to 85°C while a cryostat 21 maintains the cooled vessel 20 at less than 20°C. The inert gas is supplied via piping 31 through a mass flow controller 30, through piping 32 and dip tube 12 into heated vessel 10, through piping 33 into cooled vessel 20, and out through vent 22. The piping 31, 32, and 33 utilized in the examples was a 1/8^{th} inch stainless steel pipe. One of ordinary skill in the art would recognize that other piping may be also be utilized without detracting from the teachings hereunder. Valves 35, 36, and 37 are located on piping 31, 32, and 33. One of ordinary skill in the art would recognize that the valves may be placed in locations suitable to control the gas flows and enable disconnect/removal of vessels.

Alternatively, the above purification methods can be combined. A BCHD sample purified by distillation or evaporation may be further purified by evaporation. The inhibitor is added to the distillate or condensate prior to the subsequent evaporation.

In addition to purifying the BCHD, the apparatus 1 shown in figure 1 provides a crude approximation of conditions in a vaporizer and therefore may also be used to evaluate the effectiveness of the additive by measuring the quantity of residue remaining in the heated vessel after evaporation of a sample of the BCHD/additive composition.

This apparatus 1 is particularly effective for the removal of residue-forming impurities. It may be less effective for the removal of volatile impurities, but in many applications these are of less concern because the volatile impurities do not result in clogging of the vapor deposition apparatus. The apparatus 1 shown in figure 1 has been found to result in loss of about 20% of the starting material, but this loss could be reduced in obvious ways, for example by coiling the tubing before the cold vessel and immersing the coil in the same cooling bath in order to improve cooling and capture of the product.

The BCHD/additive composition is subsequently stored in an inert atmosphere, in a container suitable for use in the vapor deposition process. Suitable containers include stainless steel vessels such as those routinely used for vapor deposition chemicals.

The BCHD/additive composition may also be delivered from the canister to point of use using a conventional high purity liquid chemical delivery system, such as described in the article by Girard et al., Contamination-free delivery of advanced precursors for new materials introduction in lC manufacturing, 13 Future Fab International 157-162 (July, 2002), which is well adapted to enable canister exchange without contaminating the chemical or exposing the user to the canister's contents.

### General Precautions

Some general precautions for avoiding polymerization include:
(1) All surfaces in contact with the material, for example the inner surfaces of containers, delivery lines, and components, should be as dry as possible. Any surface that will be in prolonged contact, for example the interior surface of a storage vessel, should preferably be rigorously dried by baking for several hours or overnight under vacuum.
(2) The container should be made of clean materials such as electropolished stainless steel or carefully cleaned glass. Glass containers may be cleaned by acid-washing followed by thorough rinsing and drying. Gases should be filtered and have a low moisture level (preferably < 1 ppm, in any case < 100 ppm).
(3) Use of fittings, such as valves, that generate large numbers of particles should be avoided.
(4) Leaks should be eliminated.
(5) Other precautions should be taken as needed to eliminate metal and water contamination. Trace levels of metal contaminants, such as nickel, strongly catalyze the polymerization of BCHD.

### Use of Composition

The BCHD/additive composition may be used to form an insulating film on a substrate, which may or may not already include other layers thereon, by vapor deposition processes known in the art. Exemplary, but non-limiting reference to the vapor deposition processes disclosed in US 6,312,793, US 6,479,110, US 6,756,323, US 6,953,984, US 7,030,468, US 7,049,427, US 7,282,458, US 7,288,292, and US 7,312,524 and US 2007/0057235 is incorporated herein by reference.

For example, it is anticipated that, in the method disclosed of forming a layer of carbon-doped silicon oxide on a substrate in US 2007/0057235, the compositions disclosed herein may replace the cyclic alkene composition.

Similarly, the additives disclosed herein may be combined with the second precursor (also referred to as the hydrocarbon molecules or organic molecules) disclosed in US 6,312,793, US 6,479,110, US 6,756,323, US 7,030,468, US 7,049,427, US 7,282,458, US 7,288,292, and US 7,312,524 prior to the vapor deposition methods disclosed therein to prevent polymerization of the second precursor during delivery. Common highlights of these processes are further described herein.

The substrate is placed in the reaction chamber of a vapor deposition tool. The precursor(s) used to form the insulating film, non-limiting examples include those disclosed in the incorporated prior art, and the BCHD/additive composition may be delivered directly as a gas to the reactor, delivered as a liquid vaporized directly within the reactor, or transported by an inert carrier gas including, but not limited to, helium or argon. Preferably, the BCHD/additive composition is vaporized at a temperature between about 70°C and about 110°C in the presence of a carrier gas prior to introduction into the reaction chamber.

The type of substrate upon which the insulating layer will be deposited will vary depending on the final use intended. In some embodiments, the substrate may include doped or undoped silicon optionally coated with a silicon oxide layer, in addition to oxides which are used as dielectric materials in MIM, DRAM, FeRam technologies or gate dielectrics in CMOS technologies (for example, SiO₂, SiON, or HfO₂ based materials, TiO₂ based materials, ZrO₂ based materials, rare earth oxide based materials, ternary oxide based materials, etc.), and metals that are used as conducting materials in such applications, such as for example, tungsten, titanium, tantalum, ruthenium, or copper. In other embodiments, the substrate may include copper interconnects and insulating regions, such as another low-k material, optionally coated with a sealing layer such as SiO₂ or SiN.

Other examples of substrates upon which the insulating film may be coated include, but are not limited to, solid substrates such as metal substrates (for example, Ru, Al, Ni, Ti, Co, Pt and metal silicides, such as TiSi₂, CoSi₂, and NiSi₂); metal nitride containing substrates (for example, TaN, TiN, WN, TaCN, TiCN, TaSiN, and TiSiN); semiconductor materials (for example, Si, SiGe, GaAs, InP, diamond, GaN, and SiC); insulators (for example, SiO₂, Si₃N₄, HfO₂, Ta₂O₅ ZrO₂, TiO₂, A1₂O₃, and barium strontium titanate); or other substrates that include any number of combinations of these materials. The actual substrate utilized will also depend upon the insulating layer utilized.

The precursor(s) used to form the insulating film and the BCHD/additive composition are introduced into the film deposition chamber and contacted with the substrate to form an insulating layer on at least one surface of the substrate.

The film deposition chamber may be any enclosure or chamber of a device in which deposition methods take place, such as, without limitation, a parallel plate-type reactor, a cold-wall type reactor, a hot-wall type reactor, a single-wafer reactor, a multi-wafer reactor, or other such types of deposition systems.

As discussed in more detail in the incorporated prior art, the insulating layer may subsequently be rendered porous by additional processing to reduce the dielectric constant of the insulating layer. Such processing includes, but is not limited to, annealing, UV light, or electron beam.

Based on the disclosure herein and in the references incorporated by reference, one of ordinary skill in the art would be able to easily select appropriate values for the process variables controlled during deposition of the low-k films, including RF power, precursor mixture and flow rate, pressure in reactor, and substrate temperature.

### Examples:

The following examples illustrate experiments performed in conjunction with the disclosure herein. The examples are not intended to be all inclusive and are not intended to limit the scope of disclosure described herein.

### Example 1

As discussed in the background, BCHD is usually supplied with a BHT inhibitor to prevent polymerization. BHT has a vapor pressure of less than 0.01 ^{*}133 Pa at 20°C and a boiling point of 265°C.

Approximately 12 cc of purified BCHD was added to six stainless steel tubes having a 12 mm inner diameter and a 150 mm length. BHT was added to four of the tubes in concentrations of 100 ppmw, 250 ppmw, 500 ppmw, and 1000 ppmw, respectively.

All six tubes were filled under argon. One of the tubes containing only BCHD remained at 20°C to serve as a control. The remaining five tubes were placed in a silicon oil bath at 128°C for seven days. The table below provides the GC-FID analysis results after seven days. As the table below indicates, GC-FID analysis reveals no significant difference in BCHD content with or without BHT.

Other techniques are necessary, such as direct residue measurement, to assess polymerization in BCHD.

| **Sample** | **BCHD** (Conc. %) | **Light Compounds** (Conc. %) | **Benzene** (Conc. %) | **2-Norbornene** (Conc. %) | **Other** (Conc.%) |
|---|---|---|---|---|---|
| Control | 99.50 | <0.05 | ∼0.05 | ∼0.40 | <0.05 |
| Heated Control | 99.36 | <0.05 | ∼0.05 | ∼0.40 | ∼0.14 |
| 100 ppm BHT | 99.34 | <0.05 | ∼0.05 | ∼0.40 | ∼0.16 |
| 250 ppm BHT | 99.28 | <0.05 | ∼0.05 | ∼0.40 | ∼0.22 |
| 500 ppm BHT | 99.36 | <0.05 | ∼0.05 | ∼0.40 | ∼0.14 |
| 1000 ppm BHT | 99.34 | <0.05 | ∼0.05 | ∼0.40 | ∼0.16 |

### Example 2

In order to examine the effect of various gas environments on polymerization, 150 mL of distilled BCHD having a purity of 99.4% was added to the heated vessel 10 of apparatus 1 of figure 1. Gas having a flow rate of 0.5 L/min was passed through the apparatus 1. In the case of CO₂, testing was done with gas bubbling through the liquid BCHD as well as flowing over the liquid. In all other cases gas flowed over the liquid. The heated vessel 10 was maintained at 85°C by a silicone oil bath 11. Cryostat 21 maintained the cooled vessel 20 at 3°C. The total residue percentage reflects the amount of non-volatile residue remaining in the heated vessel 10 after 2.5 hours. CO₂ gas treatment was found to have a slight negative effect in the flowing condition. In the bubbling condition, the negative effect was reduced, possibly due to evaporation taking place more quickly. The effect of oxygen in increasing polymerization of BCHD was confirmed.

| **Gas** | **Total Residue (%)** |
|---|---|
| N₂ | 0.07 |
| 5% O₂ in N₂ | 0.33 |
| CO₂ | 0.10 |
| CO₂ bubbling | 0.06 |

### Example 3

To determine which compounds exhibited suitable volatility and inhibiting action, 150 mL of distilled BCHD having a purity of 99.4% was mixed with the indicated concentration of each of the additives listed below and added to the heated vessel 10 of apparatus 1 of figure 1. Nitrogen having a flow rate of 0.5 L/min was passed through the apparatus 1.

The heated vessel 10 was maintained at 85°C by a silicone oil bath 11. Cryostat 21 maintained the cooled vessel 20 at 3°C. The total residue percentage reflects the amount of residue remaining in the heated vessel 10 after 2.5 hours.

The lowest measurable residue was approximately 0.01 %, due to the difficulty of weighing the vessel from which evaporation occurred. Thus the lowest quantities of residue reported in the table below were at the method detection limit.

In some cases, the amount of residue was not weighed but was estimated from the quantity of residue visible in the glass evaporation vessel.

For additives of low volatility (e.g. PBN, 0.005*133 Pa at 25°C, and BHT, 0.010*133 Pa at 20°C), the additive itself was presumed not to evaporate. The weight of additive present was therefore subtracted from the total residue in order to calculate the residue due to BCHD itself (identified as "BCHD Residue").

The results indicate that PBN (a nitrone), TEMPO, and benzoquinone give the lowest amounts of BCHD residue, whereas several of the other additives resulted in a higher concentration of residue than BCHD alone, indicating that they did not inhibit polymerization and may have acted as initiators (e.g. acetyl acetone, limonene oxide).

| **Additive** | **Concentration added (ppmw)** | **Total Residue (%)** | **BCHD Residue (%)** |
|---|---|---|---|
| None | NA | 0.07 | 0.07 |
| Ascorbic acid | 500 | 0.25 | 0.10 |
| BHT | 500 | 0.13 | 0.08 |
| Isobutyl methacrylate | 500 | >0.2 (visual estimate) | >0.1 |
| Limonene oxide | 500 | 0.18 | 0.13 |
| N-tert-butyl-α- phenylnitrone (PBN) | 500 | 0.06 | 0.01 |
| Hexamethyldisilane | 500 | 0.07 | 0.07* |
| TEMPO | 500 | 0.02 | 0.02* |
| Acetyl acetone | 500 | 0.39 | 0.39* |
| Benzoquinone | 220 | ∼0.01 (visual estimate) | ∼ 0.01 |

| | | | |
|---|---|---|---|
| * - Identical to total residue because additives are volatile. | | | |

### Example 4

To determine the most effective concentrations of 5,5-dimethyl-1-pyrroline N-oxide (DMPO) and benzoquinone, the quantity of distilled BCHD cited below and having a purity of approximately 99.9% was mixed with the cited quantity of DMPO, benzoquinone, and a combination of benzoquinone and TEMPO and added to the heated vessel 10 of apparatus 1 of figure 1. The purity of the BCHD (99.9% compared to 99.4% in Example 3) was achieved by optimizing purification conditions of pressure, temperature, and rate of product delivery, as is known in the art. This resulted in a reduction in the level of residue obtained on evaporation of BCHD before addition of an inhibitor, from 0.07% in Example 3 to 222 ppmw or approximately 0.02%.

Nitrogen having a flow rate of 0.5 L/min was passed through the apparatus 1. The heated vessel 10 was maintained at 85°C by a silicone oil bath 11. Cryostat 21 maintained the cooled vessel 20 at 3°C. The total residue reflects the amount of non-volatile material in ppmw remaining in the heated vessel 10 after 3 hours.

The precision of weighing the residue was also substantially improved over Example 3. This was achieved by dissolving the residue in a solvent, transferring the solution to a light weighing dish, allowing the solvent to re-evaporate, and weighing the residue in the dish. A control experiment, evaporating the same quantity of solvent without any dissolved residue, indicated that the precision of residue measurement with this improved technique was about 1 ppm.

The results indicate that all of the tested inhibitors provide substantial reductions in residue compared to the sample without any inhibitor. 5 ppmw residue levels were eventually achieved using 150 ppmw benzoquinone inhibitor.

An equivalent level (within experimental error) of 7 ppmw was achieved using a combination of 150 ppmw TEMPO + 150 ppmw benzoquinone. In earlier tests (data at the top of the table) higher residue levels were obtained for about the same benzoquinone concentration, although the same careful procedures, following precautions indicated earlier, were implemented. The observed drop in residue is probably due to "clean-up" of the purification apparatus, i.e. removal of trace non-volatile contaminants through use for purification. Similar improvement is expected to be achievable for DMPO.

| **Conc. (ppmw)** | **Starting vol. of BCHD (mL)** | **Residue (ppmw)** |
|---|---|---|
| 0 | 150 | 222 |
| 150 ― benzoquinone | 150 | 21 |
| 200 ― benzoquinone | 150 | 57 |
| 1,000 ― benzoquinone | 135 | 82 |
| 150 ― DMPO | 170 | 47 |
| 300 ― DMPO | 155 | 31 |
| 1,000 ― DMPO | 150 | 26 |
| 150 ― benzoquinone | 145 | 5 |
| 150 ― benzoquinone | 155 | 5 |
| 150 ― benzoquinone | 155 | 5 |
| 1,000 ― benzoquinone | 150 | 26 |
| 150 ― benzoquinone + 150 - TEMPO | 150 | 7 |

### Example 5

The BCHD/benzoquinone composition was tested in a commercially available vaporizer. The vaporizer was operated at 85°C and 80^{*}133 Pa with a helium flow rate of 400 sccm. 406 g of the liquid BCHD/benzoquinone composition was delivered over a 5 hours period by the vaporizer, which was set at the rate of 2 g/min. The composition delivery was cycled between 3 minutes on and 2 minutes off. When the simulation was concluded, the vaporizer was disassembled to determine if any residue remained in the vaporizer. A small amount of thin film, which was insufficient to interfere with flow through the vaporizer, was detected.

It will be understood that many additional changes in the details, materials, steps, and arrangement of parts, which have been herein described and illustrated in order to explain the nature of the invention, may be made by those skilled in the art within the principle and scope of the invention as expressed in the appended claims. Thus, the present invention is not intended to be limited to the specific embodiments in the examples given above and/or the attached drawings.

## Claims

1. A composition for providing a low-k film comprising bicyclo[2.2.1]hepta-2,5-diene and at least one additive selected from the group consisting of nitrones, quinones, mixtures of nitrones and quinones, mixtures of nitrones and stable nitroxides, and mixtures of quinones and stable nitroxides, the composition containing less than 50 ppmw non-volatile material.

2. The composition of claim 1, wherein the at least one additive comprises a quinone, the composition further comprising an electron donor species.

3. The composition of claim 1, wherein the additive has a vapor pressure within a range of approximately 0.05*133 Pa to approximately 50*133 Pa at 20°C, preferably wherein the vapor pressure ranges from approximately 0.08*133 Pa to approximately 10*133 Pa at 20°C.

4. The composition of claim 1, wherein the additive comprises 5,5-dimethyl-1-pyrroline N-oxide (DMPO) and wherein the additive is present in an amount between approximately 300 ppmw to approximately 1,000 ppmw.

5. The composition of claim 3, wherein the additive comprises benzoquinone and wherein the additive is present in an amount between approximately 100 ppmw to approximately 500 ppmw.

6. The composition of claim 3, wherein the additive comprises a mixture of benzoquinone and TEMPO and wherein the mixture comprises an amount of benzoquinone between approximately 1 ppmw to approximately 150 ppmw and of TEMPO between approximately 100 ppmw to approximately 200 ppmw.

7. A method for stabilizing bicyclo[2.2.1]hepta-2,5-diene (BCHD), comprising the steps of providing BCHD and adding to the BCHD at least one additive selected from the group consisting of nitrones, quinones, a mixture of nitrones and quinones, a mixture of nitrones and stable nitroxides, and a mixture of quinones and stable nitroxides.

8. The method of claim 7, wherein the at least one additive comprises a quinone, further comprising adding an electron donor species to the quinone.

9. The method of claim 7, further comprising purifying the BCHD, wherein the additive is added within one hour of purification.

10. The method of claim 9, wherein the BCHD is purified to at least 99.0%.

11. The method of claim 10, wherein an amount between approximately 300 ppmw and approximately 1,000 ppmw of 5,5-dimethyl-1-pyrroline N-oxide is added to the BCHD and preferably wherein an amount between approximately 100 ppmw and approximately 500 ppmw of benzoquinone is added to the BCHD.

12. The method of claim 10, wherein the mixture containing an amount between approximately 1 ppmw and approximately 150 ppmw of benzoquinone and between approximately 100 ppmw and approximately 200 ppmw of TEMPO is added to the BCHD.

13. A method of forming an insulating film layer on a substrate, the method comprising the steps of:
- providing a reaction chamber having at least one substrate disposed therein;
- introducing at least one precursor compound into the reaction chamber;
- introducing into the reaction chamber a composition comprising bicyclo[2.2.1]hepta-2,5-diene and at least one additive selected from the group consisting of nitrones, quinones, a mixture of nitrones and quinones, a mixture of nitrones and stable nitroxides, and a mixture of quinones and stable nitroxides, the composition further comprising an electron donor species; and
- contacting the at least one precursor compound, the composition, and the substrate to form an insulating film on at least one surface of the substrate using a deposition process.

14. The method of claim 13, further comprising vaporizing the composition at a temperature between about 70°C and about 110°C in the presence of a carrier gas prior to introduction into the reaction chamber.

15. The method of claim 13, wherein the additive comprises between approximately 300 ppmw and approximately 1,000 ppmw of 5,5-dimethyl-l-pyrroline N-oxide, preferably wherein the additive comprises between approximately 100 ppmw and approximately 500 ppmw of benzoquinone and more preferably wherein the additive comprises the mixture of approximately 1 ppmw to approximately 150 ppmw of benzoquinone and between approximately 100 ppmw to approximately 200 ppmw of TEMPO.
